# EUROPEAN PATENT SPECIFICATION

(11) **EP 1 052 956 B1**
(45) Date of publication and mention of the grant of the patent: **02.06.2004**
(21) Application number: 99905343.2
(22) Date of filing: 12.02.1999
(51) Int. Cl.: A61F 5/455

(54) **A URINE DISCHARGE APPARATUS FOR FEMALE**
VORRICHTUNG ZUM AUFFANG VON URIN FÜR FRAUEN
APPAREIL D'EVACUATION D'URINE POUR LES FEMMES

(30) Priority: 13.02.1998 KR 9804280; 27.01.1999 KR 9902595
(43) Date of publication of application: 22.11.2000
(60) Divisional application: 03022211.1
(73) Proprietor: Kim, Kyoung-Hun, Pusan 602-030 (KR)
(72) Inventor: Kim, Kyoung-Hun, Pusan 602-030 (KR)
(74) Representative: Urner, Peter, Dipl.-Phys.
(86) International application number: PCT/KR1999/000074
(87) International publication number: WO 1999/040879

(56) References cited:
- DE-A1- 3 042 451
- DE-C2- 4 411 824
- GB-A- 2 091 560
- US-A- 5 004 463

## Description

The present invention relates to a urine discharge apparatus for female, and particularly to the urine discharge apparatus providing advantage that an urine collected in disposable pack of collect member is discharged without detention by use of a small pump and it is convenient to carry and keep due to its small volume in being folded. Generally, urination of female is different from that of male, and has a problem that female should expose excessively her body for maintaining a sitting position with her trouser taken off. In order to hide the exposed body, female usually use a toilet seats in a closed space. However, in a case that a public toilet is not available at outdoor, urination of female is inevitably accompanied by an excessive exposure of body which impose a burden on her and may evoke a gender complex.

In order to minimize the excessive exposure of body and overcome the complex, "female urine discharge device" disclosed by Publication of Laid-open Korea Patent Application 95-5332 was proposed.

The proposed device has a funnel-shaped hemisphere and protects leakage of urine by contact portion of the hemisphere whose rim was made of soft material, a saw-toothed anti-back current means in interior surface for preventing back current of urine after urination and a outlet pipe that is wrinkled.

While not used, this device is kept in a pocket placed at underwear in a manner that the outlet pipe is attached to underwear and folded upwardly.

Since the contact portion is easily separated from the hemisphere when the device is taken out from the pocket or used, however, the device cause much inconvenience and the underwear may be spoiled by spreading of urine because urethra of female is placed at lower portion of body and thus urine is not collected in outlet pipe protruding forward. Accordingly, a repeated use of the device is impossible and cumbersomeness that female always wear during her activity is accompanied.

In addition, since female usually urinate 7 or 8 times and deject 1 or 2 times a day, female use a toilet seat more frequently than male and thus public toilet may be overcrowded.

Accordingly infection through toilet seat may take place more frequently.

The GB 2091560 A discloses a female underpant with urination facility. Female underpants are formed from a material which is watertight and adherent to the body of the wearer. The crotch of the underpants is provided with a cavity facing the urinating organ of the female body and has a urine discharge port projecting from the lower end of the cavity. To this urine discharge port is detachably connected a urine discharge device provided with a pump so that the urine voided into the cavity can be discharged by forced suction produced by the urine discharge device, thereby enabling a female driver or passenger in a car to urinate with the underpants on when no lavatory is conveniently available.

The present invention is proposed to solve the above-mentioned problem, and the object of the invention is to provide "urine discharge apparatus for female" that female carry the apparatus at normal time and attach the apparatus on perineum as pose of male urinating in the time of urination, the apparatus is also convenient to carry and keep and make it possible to examine the amount of urine and so on and therefore has a medical effect with early detection of disease.

After urination, the urine collected in the collect member is discharged outside without detention by pump.

The present invention also provide urine discharge apparatus for female whose driving member, pump member and collect member can cooperate one another by hinge coupling so as to control easily the direction of urine discharged.

Another object of the present invention is to provide a urine discharge apparatus for female that use a disposable pack for sanitary urination, the disposable pack is also used for medical examination and pregnancy diagnosis.

Also, another object of the present invention is to provide a urine discharge apparatus for female that enable a bedridden patient to urinate by oneself or with help of nurse in the bed.
Fig.1 is an exploded perspective view showing a condition of the separated urine discharge apparatus according to one embodiment of the present invention,

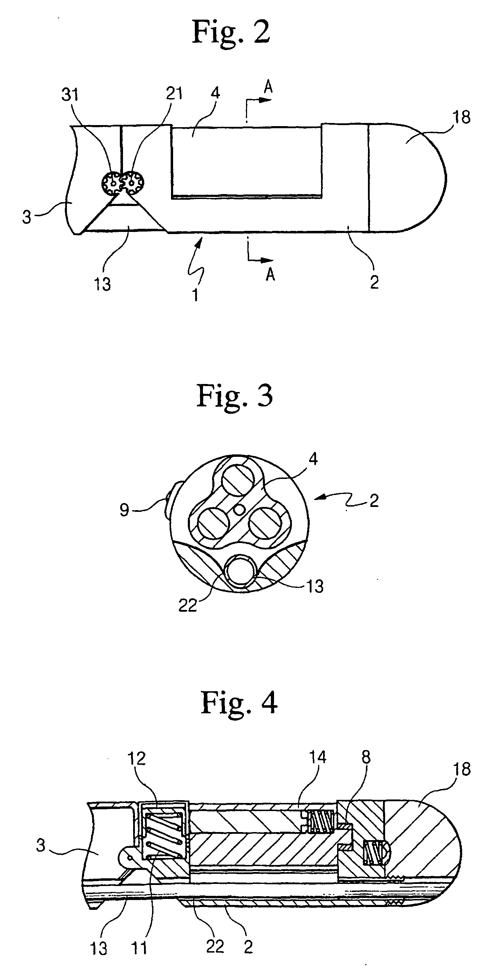

Fig. 2 is a side view showing a part of urine discharge according to one embodiment of the present invention,
Fig. 3 is a cross-sectional view taken from line A-A of the Fig. 2,
Fig. 4 is a longitudinal sectional view of the Fig. 2 ,
Fig. 5 is a side view showing a operational condition of urine discharge apparatus according to one embodiment of the present invention,
Fig. 6 is a side view showing a using condition of the urine discharge apparatus according to one embodiment of the present invention,
Fig. 7 is a side view showing a condition that a auxiliary hose is connected to top end of the discharge hose according to one embodiment of the present invention,
Fig. 8 is a modification of one embodiment, a partly sectional side view showing a condition that additional float-type switch for applying electric power is provided,

The description of reference numbers is as follows:
- 1:: body
- 2:: driving member
- 3:: pump member
- 4:: receptacle
- 5:: pump
- 6:: collect member
- 7:: disposable pack
- 9:: switch
- 12:: door
- 13:: discharge hose
- 13':: auxiliary hose
- 15:: discharge pipe
- 16:: test paper
- 41, 42:: receptacle
- 51:: impeller
- 52:: entrance
- 53:: outlet
- 62:: magnet
- 81:: groove
- 82:: rear wall
- 83:: clip
- 831:: blade portion
- 85:: bottom
- 87:: displayer
- 88:: holder
- 131:: introduce hose

According to the present invention, urine discharge apparatus for female comprises a body which is divided into a driving member and a pump member and these member cooperate one another by hinge means of gear, a battery case that is rotatably connected to holding clip mounted on the driving member and receive a battery during rotation to supply power, a switch which is located on outer surface of the driving member and switch the electric power on and off, a door that is incorporated in driving member and open and close an entrance of the battery case by spring therein, a pump which is incorporated in pump member and has a impeller inside thereof and a inlet and outlet for urine passage at both side, a collect member that is connected to one side of the pump member by hinge means and collect urine, a discharge pipe which is connected to the midpoint of hinge means coupling the pump member and the collect member and introduce the urine collected in the collect member to the inlet of pump, a discharge hose whose one end is connected to the outlet of the pump and the other communicate with through passage formed in lower portion of the driving member such that the urine can be discharged outside through discharge hose.

Also, as the above switch consists of adjustable resistance, the amount of urine discharged can vary by control of rotation of the pump.

Further, urine discharge apparatus for female according to the present invention more comprises a disposable pack which is mounted on the collect member and contact with perineum, a side wall of the disposable pack has a circumferential flange and a non-woven fabric is attached to lower surface of the disposable pack, thus spread of the urine can be prevented.

Moreover, the urine discharge apparatus according to the present invention more comprises a cover which protects the driving member and the pump member as well as present aesthetic pleasure.

Further, the disposable pack has a test paper inside thereof for health examination and testing pregnancy.

Furthermore, the urine collected in the collect member can be easily discharged by connecting auxiliary hose to top end of discharge hose inserted in through passage of the driving member.

Furthermore, since a float-type switch is additionally added to inside of the collect member, the pump is provided with electric power and automatically operated as soon as the collect member receive a urine.

The present invention will be described in further detail with reference to the accompanying drawings.

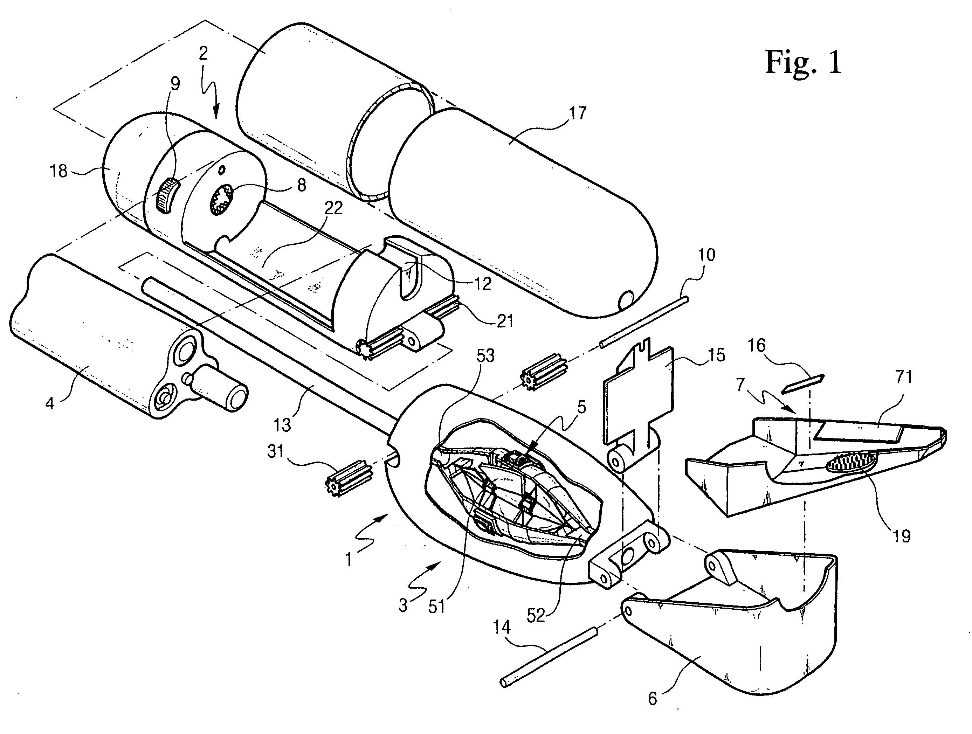

Fig. 1 is an exploded perspective view showing a condition of separated urine discharge apparatus for female according to one embodiment of the present invention, a body 1 is divided into the driving member 2 and the pump member 3, and these divided member are connected to corresponding portion by hinge means of pin 10 to cooperate each other in manner that gear 21 engages with gear 31.

The driving member 2 of the body 1 has a space for carrying the battery case 4, and the holding clip 8 is provided in the centre of both side thereof so as to restrict rotation of the receptacle 4.

And, the switch 9 for switching electric power is provided on outer surface of the driving member 2 and may be located on right or left side depending on user in order that user can operate the switch 9 with thumb, a door 12 which open and close the entrance of the battery case 4 by spring 11 is provided on opposing side of the switch 9, a through passage 22 is formed in the bottom of the driving member 2, a discharge hose 13 is inserted into through passage and protrude outward from end of the driving member 2.

The pump member 3 of the body 1 has a space for containing small pump 5 and its one side is connected to collect member 6 by hinge means of a pin 14 to cooperate with the collect member 6, the pump 5 has the discharge pipe 15 and the discharge hose 13 at both side.

The collect member 6 is connected to pump member 3 by hinge means of pin 14 and upwardly folded in the time of keeping and carrying, and may be unfolded to a predetermined angle when used.

The discharge pipe 15 provided in the middle of hinge means connecting the pump member 3 to the collect member 6 can rotate independently, a flange at both side of the discharge pipe 15 contact completely with surface of the collect member 6 to support it, and the discharge pipe 15 provide a passage for introducing the urine collected in the collect member 6 to inlet 52 of the pump 5.

The pump 5 is incorporated in the pump member 3 of the body 1 and is provided with impeller 51 at inside thereof, the impeller 51 rotate by electric power supplied through the switch 9 to discharge urine, and the pump 5 has the inlet 52 communicating with the discharge pipe 15 in one side and the outlet 53 communicating with the discharge hose 13 in the other side.

The pump 5 above mentioned is not limitative and a typical pump generally used for urine discharge, gear pump and so on, may be used.

The battery case 4 contains three 1.5 V batteries, and is fitted in the holding clip 8 for restricting the battery case 4, a positive pole of the battery is connected to the switch 9 and a negative pole to the pump 5 in normal way.

Also, a rechargeable cell and charger may be used instead of the battery.

The switch 9 operates the pump 5 by switching electric power, and may be replaced by adjustable resistance switch if control of rotation of the pump 5 is necessary.

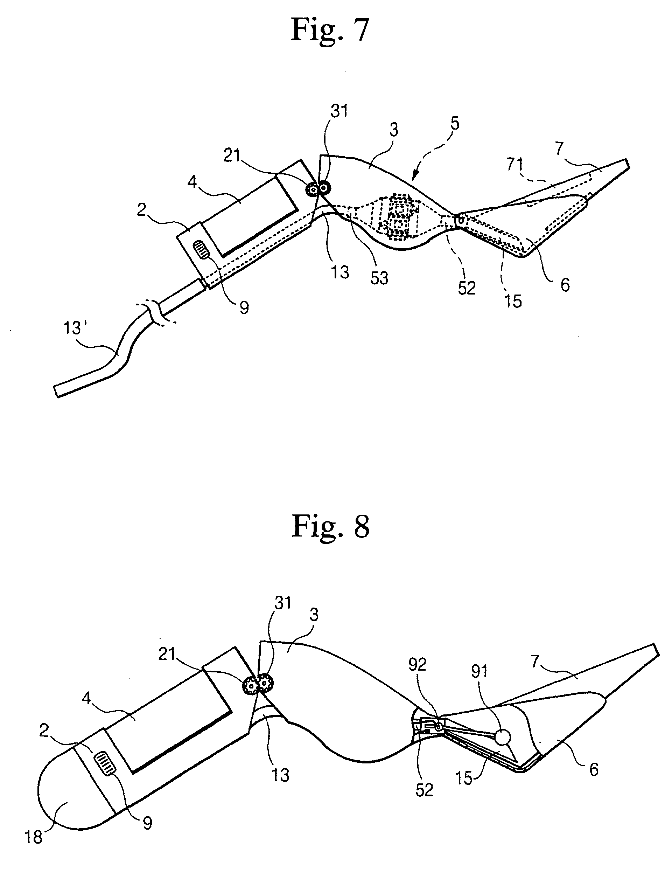

Also, as shown in Fig.8, a floating- type switch 92 may be additionally provided so as to connect parallel a floater 91 and the switch 9 of the driving member 2, the floater 91 is raised from a surface of the collect member as soon as an urine is collected in the collect member 6.

By above movement, the float-type switch 92 may be operate and supply automatically electric power to drive the pump 5.

This operation make it possible to discharge a urine independently of the switch 9 of the driving member 2.

The disposable pack 7 mounted on the collect member 6 is sold and kept in folded condition. When used, the disposable pack 7 is unfolded to obtain space for collecting a urine and attached to perineum

The flange 71 is provided longitudinally at both side wall so as to prevent the urine discharged from urethra from spreading and collect a urine easily.

Also, non-woven fabric may be attached on bottom of the disposable pack 7 to obtain the effect of absorption and prevent spread of urine.

Further, test paper 16 may be attached to inside of the disposable pack 7 in order that user, if necessary, can examine a proteinuira, a haematouria, a glycosuria and a pregnancy for private checkup.

On the other hand, the cover 17 made of resin or fabric may be used to wrap the driving member 2 of the body 1 and pump member 3 for protection and aesthetic pleasure.

As shown in Fig.7, auxiliary hose 13' may be connected to top end of the discharge hose 13 protruding outward to ensure a bedridden patient can urinate in bed with help of nurse or by oneself, and thus the discharged urine may be easily collected in other drainage or urinal.

The reference number 18 in drawings is a cap connected to the top end of driving member 2 of the body 1.

The using method of the above mentioned urine discharge apparatus will be described in followings.

The battery case 4 receive a battery while rotate repeatedly about a third of one cycle after entrance of the receptacle 4 is opened by lowering the door 12 of the driving member 2, and the driving member 2, the pump member 3 and the collect member 6 is adjusted to the same angle that the male urinate.

The collect member 6 is attached to perineum after the discharge pipe 15 contact on the surface of the collect member 6, and the pump 5 will be rotate simultaneously with urination or right before urination while the driving member 2 and the pump member 3 is adapted to convenient angle for urination.

The urine discharged from urethra is introduced to the discharge pipe 15 by sucking operation of the pump 5 as soon as collected in the collect member 6, and continuously flow from the inlet 52 of the pump 5 and impeller 51 to the outlet 53, and discharged outward by eject operation of the pump 5 through discharge hose 13.

After the urine collected in the collecting member 6 is completely discharged, the operation of the pump 5 come to a halt by the switch 9 off. Thus a operating cycle of the urine discharge apparatus is finished and the apparatus may be folded for carrying or keeping.

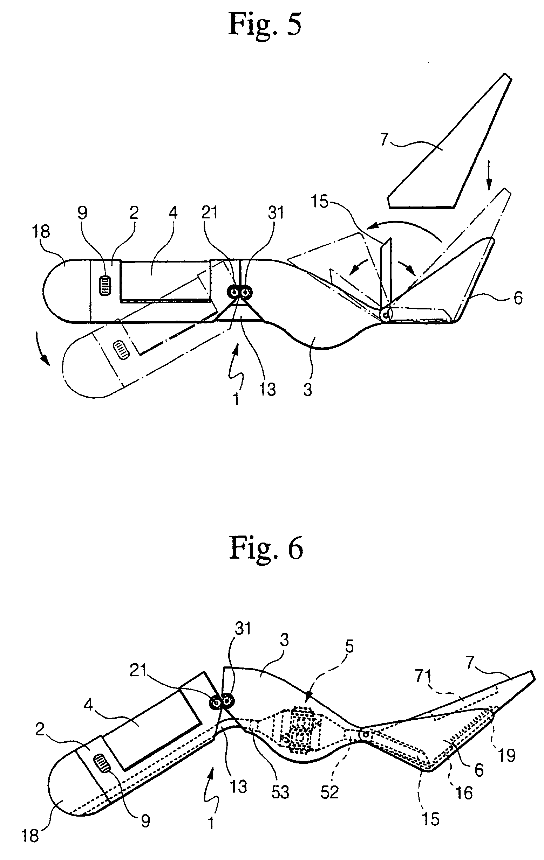

Also, in case the disposable pack 7 is used as shown in the Fig. 5 and 6, a using method of the apparatus is same with that of the above-mentioned case excepting the disposable pack 7 is compressed by the discharge pipe 15.

Further, the use of the disposable pack 7 may ensure more sanitary urination because the urine is absorbed in the non-woven fabric 19 without spread ,and the flange 71 extending longitudinally in side wall also prevent effectively spread of the urine.

Further, the test paper 16 attached to inside of the disposable pack 7 enable user to check easily health index.

## Claims

1. A urine discharge apparatus for a female comprising:
- a collection member (6) and a pump member (3) connected to the collection member (6) and including a pump (5) inside thereof;
- the pump (5) is open to the collection member (6), wherein the pump (5) is provided for removing urine collected in the collection member (6) and discharging the urine into an outlet (53) thereof; and
- a driving member (2), which is connected to the pump member (3)
**characterized in that,**
- the collection member (6) has the shape of a bucket; and the pump member (3) is connected to the collection member (6) by a hinge coupling; and
- the driving member (2) is connected to the pump member (3) by hinge coupling and contains a battery (4), wherein the battery supplies electric power for operating the pump (5).

2. The urine discharge apparatus as claimed in claim 1, wherein said driving member (2) has a battery case (4) which is axially connected to a holding clip (8) and receiving a battery upon rotation away from the driving member (2) and a switch (9) which is mounted on a top end of the driving member (2) for turning on the electric power, and a door (12) which is provided in an end portion of said driving member (2) provided to open and close the entrance of the battery case (4) by action of a spring.

3. The urine discharge apparatus as claimed in claim 1, wherein the driving member (2) includes a through passage (22) formed longitudinally in a bottom portion of the driving member (2) such that a discharge hose (13) extending from the outlet (53) of the pump (5) penetrates the driving member (2) within the through passage (22).

4. The urine discharge apparatus as claimed in claim 1, wherein the collection member (6) includes a disposable pack (7) mounted on the collection member (6), the disposable pack (7) having a flange (71) on a side wall adapted for contacting the perineum.

5. The urine discharge apparatus for a female as claimed in any one of claim 1 to 4, further comprising a discharge pipe (15) coupled in the middle of a hinge for connecting the collection member (6) and the pump member (3) for introducing the urine collected in the collection member (6) to an inlet (52) of the pump (5) and compress the disposable pack (7) to a bottom surface of the collection member (6).

6. The urine discharge apparatus for a female according to claim 2, wherein the switch is an adjustable resistance switch for controlling rotation speed of the pump (5).

7. The urine discharge apparatus for a female according to claim 4, wherein a test paper (16) is attached within the disposable pack (7) for health and pregnancy testing.

8. The urine discharge apparatus for a female according to claim 4, wherein a non-woven fabric (19) is attached within the disposable pack (7) for preventing urine from leaking.

9. The urine discharge apparatus for a female as claimed in any one of claim 1 to 3, further comprising an auxiliary hose (13') connected to an end of the discharge hose (13) such that the auxiliary hose ( 13') protrudes outward from the driving member (2).

10. The urine discharge apparatus for a female according to claim 2, wherein a float-type switch (92) is provided within the collection member (6).

11. The urine discharge apparatus for a female according to claim 1, further comprising a cover (18) for protecting the driving member (2) and the pump member (3) of the body.

## Patentansprüche

1. Urinauffangvorrichtung für Frauen, mit:
- einem Sammelelement (6) und einem mit diesem verbundenen Pumpenelement (3) mit einer Pumpe (5) in seinem Inneren;
- wobei die Pumpe (5) zum Sammelelement (6) hin offen ist und sie vorhanden ist, um im Sammelelement (6) gesammelten Urin zu entnehmen und ihn in einen zugehörigen Auslass (53) auszugeben; und
- einem Antriebselement (2), das mit dem Pumpenelement (3) verbunden ist;
**dadurch gekennzeichnet, dass**
- das Sammelelement (6) die Form eines Eimers hat und das Pumpenelement (3) über eine Scharnierverbindung mit dem Sammelelement (6) verbunden ist; und
- das Antriebselement (2) durch eine Scharnierverbindung mit dem Pumpenelement (3) verbunden ist und es eine Batterie (4) enthält, die elektrische Energie zum Betreiben der Pumpe (5) liefert.

2. Urinauffangvorrichtung nach Anspruch 1, bei der das Antriebselement (2) ein Batteriegehäuse (4), das axial mit einer Halteklammer (8) verbunden ist und beim Wegdrehen vom Antriebselement (2) eine Batterie aufnimmt, und einen Schalter (9), der am oberen Ende des Antriebselements (2) angebracht ist, um die elektrische Spannung einzuschalten, sowie eine Tür (12) aufweist, die in einem Endabschnitt des Antriebselements (2) vorhanden ist, um den Eingang des Batteriegehäuses (4) durch die Wirkung einer Feder zu öffnen und zu schließen.

3. Urinauffangvorrichtung nach Anspruch 1, bei der das Antriebselement (2) über einen Durchgangskanal (22) verfügt, der in der Längsrichtung in einem Bodenabschnitt desselben so ausgebildet ist, dass ein sich ausgehend vom Auslass (53) der Pumpe (5) erstreckender Auslassschlauch (13) das Antriebselement (2) innerhalb des Durchgangskanals (22) durchdringt.

4. Urinauffangvorrichtung nach Anspruch 1, bei der das Sammelelement (6) über eine an ihm angebrachte Wegwerfeinlage (7) verfügt, die an einer Seitenwand, die für Kontakt mit dem Damm ausgebildet ist, einen Flansch (71) aufweist.

5. Urinauffangvorrichtung für Frauen nach einem der Ansprüche 1 bis 4, ferner mit einer Auslassleitung (15), die in der Mitte eines Scharniers zum Verbinden des Sammelelements (6) und des Pumpenelements (3) verbunden ist, um den im Sammelelement (6) gesammelten Urin in einen Einlass (52) der Pumpe (5) einzuleiten und die Wegwerfeinlage (7) an eine Bodenfläche des Sammelelements (6) zu drücken.

6. Urinauffangvorrichtung für Frauen nach Anspruch 2, bei der der Schalter ein solcher mit einstellbarem Widerstand zum Steuern der Drehzahl der Pumpe (5) ist.

7. Urinauffangvorrichtung für Frauen nach Anspruch 4, bei der innerhalb der Wegwerfeinlage (7) ein Testpapierstück (16) für Gesundheits- und Schwangerschaftstests angebracht ist.

8. Urinauffangvorrichtung für Frauen nach Anspruch 4, bei der innerhalb der Wegwerfeinlage (7) ein Vlies. (19) angebracht ist, um ein Auslecken von Urin zu verhindern.

9. Urinauffangvorrichtung für Frauen nach einem der Ansprüche 1 bis 3, ferner mit einem Hilfsschlauch (13'), der mit einem Ende des Auslassschlauchs (13) so verbunden ist, dass er vom Antriebselement (2) nach außen vorsteht.

10. Urinauffangvorrichtung für Frauen nach Anspruch 2, bei der innerhalb des Sammelelements (6) ein Schwimmerschalter (92) vorhanden ist.

11. Urinauffangvorrichtung für Frauen nach Anspruch 1, ferner mit einer Abdeckung (18) zum Schützen des Antriebselements (2) und des Pumpenelements (3) des Körpers.

## Revendications

1. Appareil d'évacuation d'urine pour les femmes comprenant :
- un élément de collecte (6) et un élément de pompage (3) relié à l'élément de collecte (6) et renfermant une pompe (5) ;
- la pompe (5) communiquant avec l'élément de collecte (6) de façon à extraire l'urine collectée dans l'élément de collecte (6) et comprenant une sortie (53) par laquelle elle évacue cette urine ; et
- un élément de commande (2) relié à l'élément de pompage (3),
l'appareil étant **caractérisé en ce que**
- l'élément de collecte (6) est en forme de godet ; et l'élément de pompage (3) est relié à l'élément de collecte (6) par une liaison charnière ; et
- l'élément de commande (2) est relié à l'élément de pompage (3) par une liaison charnière et contient une pile ou batterie, cette pile alimentant la pompe (5) en énergie électrique.

2. Appareil d'évacuation d'urine selon la revendication 1, dans lequel ledit élément de commande (2) dispose d'un compartiment à piles (4) relié axialement à une pince de fixation (8) et pouvant tourner à l'écart de l'élément de commande (2) pour accueillir une pile, ainsi que d'un interrupteur (9) monté sur une extrémité supérieure de l'élément de commande (2) pour commander l'alimentation électrique, et d'une porte (12) située dans une extrémité dudit élément de commande (2) de façon à ouvrir ou fermer l'entrée du compartiment à piles (4) sous l'action d'un ressort.

3. Appareil d'évacuation d'urine selon la revendication 1, dans lequel l'élément de commande (2) comprend un passage traversant (22) formé longitudinalement dans une partie basse de l'élément de commande (2) permettant à un tuyau d'évacuation (13) venant de la sortie (53) de la pompe (5) de pénétrer dans l'élément de commande (2) par ledit passage traversant (22).

4. Appareil d'évacuation d'urine selon la revendication 1, dans lequel l'élément de collecte (6) comprend, montée sur lui, une garniture jetable (7) munie, sur une paroi latérale, d'un rebord (71) adapté à un contact avec le périnée.

5. Appareil d'évacuation d'urine selon l'une des revendications 1 à 4, comprenant en outre un conduit d'évacuation (15) couplé au milieu d'une charnière reliant l'élément de collecte (6) et l'élément de pompage (3), de façon à introduire dans une entrée (52) de la pompe (5) l'urine collectée dans l'élément de collecte (6) et à comprimer la garniture jetable (7) sur une surface du fond de l'élément de collecte (6).

6. Appareil d'évacuation d'urine selon la revendication 2, dans lequel l'interrupteur est un interrupteur à résistance réglable commandant la vitesse de rotation de la pompe (5).

7. Appareil d'évacuation d'urine selon la revendication 4, dans lequel un papier test (16), de grossesse ou de santé, est fixé à l'intérieur de la garniture jetable (7).

8. Appareil d'évacuation d'urine selon la revendication 4, dans lequel un textile non tissé (19) est fixé à l'intérieur de la garniture jetable (7) de façon à éviter les fuites d'urine.

9. Appareil d'évacuation d'urine selon l'une des revendications 1 à 3, comprenant en outre un tuyau auxiliaire (13') raccordé à une extrémité du tuyau d'évacuation (13) de façon à ce que le tuyau auxiliaire (13') dépasse vers l'extérieur de l'élément de commande (2).

10. Appareil d'évacuation d'urine selon la revendication 2, dans lequel un interrupteur à flotteur (92) est disposé à l'intérieur de l'élément de collecte (6).

11. Appareil d'évacuation d'urine selon la revendication 1, comprenant en outre un couvercle (18) pour protéger l'élément de commande (2) et l'élément de pompage (3).
